# EUROPEAN PATENT APPLICATION

(11) **EP 2 808 010 A1**
(43) Date of publication of application: **03.12.2014**
(21) Application number: 14170032.8
(22) Date of filing: 27.05.2014
(51) Int. Cl.: A61K 9/00, A61K 47/38, A61K 31/137

(54) **Syrup Formulation of Salbutamol**

(30) Priority: 28.05.2013 TR 201306298
(71) Applicant: Sanovel Ilac Sanayi ve Ticaret A.S., 34460 Istanbul (TR)
(72) Inventor: Türkyilmaz, Ali, 34460 Istanbul (TR); Aygül, Fatih Cengiz, 34460 Istanbul (TR); Celik, Devrim, 34460 Istanbul (TR); Gündogdu, Yusuf, 34460 Istanbul (TR); Hatirnaz, Zekiye Basak, 34460 Istanbul (TR)
(74) Representative: Sevinç, Erkan

(57) **Abstract**

The present invention concerns an oral liquid pharmaceutical composition of salbutamol for use in the treatment of asthma, bronchospasm and reversible airways obstruction by widening the airways of the lungs.

## Description

### Technical Field

The present invention concerns an oral liquid pharmaceutical composition of salbutamol for use in the treatment of asthma, bronchospasm and reversible airways obstruction by widening the airways of the lungs.

### Background of the Invention

Salbutamol, also known as albuterol, is a selective beta-2 agonist which is useful in the treatment of asthma, bronchospasm and reversible airways obstruction. It is currently available in sulphate salt form which can be found in a variety of dosage forms including tablets, syrups and formulations suitable for inhalation.

The currently marketed syrup formulation of salbutamol sulphate is named as Ventolin® Syrup. These syrup formulations are advantageous because they are easy to administer and suitable for certain patient populations, for example children or adults who are unable to swallow solid oral dosage forms or use an inhaler device. However, as well as advantages, syrup formulations have their own problems like adjustment of desired stability, solubility, viscosity and pH.

Enhancing the stability of the active ingredient both short term and over time, is a prime goal in formulating liquid pharmaceutical compositions. Especially, if the formulation contains water, active ingredients are less stable than in the solid dosage form. As well as stability, the solubility of the active ingredient present in liquid dosage forms is also important to achieve the desired concentration of drug in the treatment. Furthermore, solubility is one of the factors affecting stability of the active ingredient. Stability can be increased by enhancing solubility.

In prior art, some studies exist to formulate salbutamol more stable in liquid oral dosage forms. U.S Pat. No. 4,594,359 (the '359 patent) discloses salbutamol aqueous formulations using cellulose derivatives to provide the desired stability and viscosity for oral use. Here, as seen in examples of the patent, the liquid formulation contains only 22.5 mg of cellulose derivative to stabilize the formulation. Another patent of salbutamol syrup formulations numbered as U.S Pat. No. 4,499,108 (the '108 patent) is about a formulation claiming pleasant taste and enhanced stability by using together buffer, preservative and saccharin. Both '108 and '359 patent discloses a process to manufacture the liquid formulation of salbutamol wherein cellulose derivative is dispersed in water and then mixed with a solution of salbutamol.

However, under certain storage conditions involving contact with the air, stability problems can still occur. Therefore, there still exists a need for new stable syrup formulations for the treatment of asthma, bronchospasm and/or reversible airways obstruction which is suitable oral therapy for children and adults who are unable to use an inhaler device.

### Object of the Invention

The main object of the present invention is to obtain an oral liquid pharmaceutical composition with bronchodilator activity, which is capable of providing an easy administration accompanied by an increased patient compliance and convenience.

Another object of the present invention is to provide an oral liquid pharmaceutical composition which avoids the afore-mentioned disadvantages of the liquid formulations of the prior art and have advantages over them.

Yet another object of the present invention is to provide an oral liquid pharmaceutical composition wherein a significantly improved pharmaceutical stability, as well as the excellent long-term stability is shown.

There is also provided an oral liquid pharmaceutical composition wherein the active ingredient is dissolved properly in liquid composition. Thus, stability of active ingredient and of liquid composition in which active ingredient is dissolved, can be secured.

There is also provided an oral liquid pharmaceutical composition wherein the pH of the composition is maintained within a range optimized for solubility of the active ingredient. There is also provided a sugar-free oral liquid pharmaceutical composition with a pleasant taste wherein the bitter taste of active ingredient is masked by the presence of at least one pharmaceutically acceptable sweetening agent suitable for the administration to diabetics. Thus, pharmaceutical composition of the present invention has a pleasant taste that permits it to be administrated to children without any reluctance.

### Detailed Description of the Invention

In order to achieve the aforementioned objects, the present invention discloses a novel, stable, bioavailable, liquid pharmaceutical composition for the oral administration of salbutamol as the active ingredient comprising one or more pharmaceutically acceptable excipient.

The term "salbutamol" as used herein refers to salbutamol as well as pharmaceutically acceptable salts, enantiomers, hydrates, solvates, metabolites, prodrugs and mixture thereof. The term also includes all polymorphic forms, whether crystalline or amorphous. According to the present invention, the preferred form of salbutamol is salbutamol sulphate.

According to an embodiment of the present invention, said oral liquid pharmaceutical composition contains salbutamol present in a concentration of about 0.1 to 5 mg/mL and preferably in a concentration of about 0.4 to 1 mg/mL.

According to a preferred embodiment, the present invention concerns an oral liquid pharmaceutical composition comprising a) salbutamol as an active agent or a pharmaceutically acceptable salt form and b) a thickening agent as an excipient which can be present in a concentration of about 20 to 80 mg/mL. In prior art, thickening agent used in salbutamol liquid formulations is in a concentration of 0.2 mg/mL. We have now unexpectedly and surprisingly discovered that the increased amount of thickening agent compared to prior art provides superior storage stability. As a further embodiment of the present invention, thickening agent used in the liquid composition has a viscosity within the range of from 1 to 10 centipoises (cP) as determined by the well-known method named "Capillary Method". We have also found that the use of a thickening agent with low viscosity changing whithin the range suitable for the present invention provides a reduced processing time. Because, thickening agents of the invention have a greater solubility of water and they provide active ingredient to be dissolved rapidly and to be more stable in the liquid pharmaceutical composition. In conjunction with the rapid dissolution of active ingredient, the manufacturing process is completed quickly, the duration of pharmaceutical formulation contacting with air is reduced and the formulation's stability is enhanced.

According to more preffered embodiment, the thickening agent can be present in a concentration of about 30 to 50 mg/mL and preferably in a concentration of about 40 mg/mL. As a further preffered embodiment, the liquid composition has a viscosity within the range of from 3.5 to 6.5 cP.

According to an embodiment, thickening agent of the present invention is selected from the group comprising, but not limited to, cellulose derivatives, guar gum, gelatin, locust bean gum, tara gum, xanthan gum, tamaring gum, povidone, aluminium silicate, magnesium silicate, propylene glycol alginate, sodium carboxymethylcellulose, sodium alginate, pectin, carrageenan, polyethylene glycol, modified starch and the like or combination thereof. The preferred cellulose derivatives can be cellulose hydroxypropyl cellulose, hydroxypropyl methylcellulose, hydroxyethyl cellulose, methyl cellulose, microcrystalline cellulose and the like or combination thereof. The most preferred cellulose derivative of the invention is hydroxypropyl methylcellulose (HPMC). As a further embodiment of the present invention, HPMC has a viscosity value within the range 1 to 10 cP, more preferably 3.5 to 6.5 cP.

According to a preferred embodiment of the present invention, the oral liquid pharmaceutical composition comprising a) salbutamol as an active agent or a pharmaceutically acceptable salt form and b) a cellulose derivative, preferably HPMC, as thickening agent wherein the cellulose derivative is present in a concentration of about 20 to 80 mg/mL, preferably in a concentration of about 30 to 50 mg/mL and most preferably in a concentration of about 40 mg/mL and has a viscosity within the range of from 1 to 10 cP, more preferably from 3.5 to 6.5 cP.

According to more preffered embodiment, the oral liquid pharmaceutical composition comprising a) salbutamol as an active agent or a pharmaceutically acceptable salt form and b) HPMC as thickening agent wherein HPMC is in a concentration of 40 mg/mL and has a viscosity within the range of from 3.5 to 6.5 cP.

According to a preferred embodiment of the present invention, the oral liquid pharmaceutical composition further comprises a disaggregating agent which can stabilize the liquid composition against aggregation of thickening agent. In prior art, it is known that thickening agent can form agglomerates which conventional equipment cannot disperse. Here, the addition of the disaggregating agent minimizes this aggregation and provide better stability. As a further embodiment, the disaggregating agent is present in a concentration of about 0.1 to 5 mg/mL and preferably in a concentration of about 0.5 to 2 mg/mL.

Disaggregating agent used according to the present invention is selected from the group comprising, but not limited to, sodium chloride, calcium chloride, potassium chloride, calcium phosphate, calcium sulphate, starch, alginic acid, sodium starch glycolate, alginates, boric acid, sorbitol and glycerol and the like or combination thereof. The preferred disaggregating agent of the present invention is sodium chloride.

According to a preferred embodiment of the present invention, the oral liquid pharmaceutical composition comprises;
a) salbutamol as an active agent or a pharmaceutically acceptable salt form,
b) a cellulose derivative, preferably HPMC, as thickening agent wherein the cellulose derivative is present in a concentration of about 20 to 80 mg/mL, and present with a viscosity within the range 1 to 10 cP, and
c) a disaggregating agent, preferably sodium chloride, present in a concentration of about 0.1 to 5 mg/mL.

According to a more preferred embodiment of the present invention, the oral liquid pharmaceutical composition comprises;
a) salbutamol as an active agent or a pharmaceutically acceptable salt form,
b) a cellulose derivative, preferably HPMC, as thickening agent wherein the cellulose derivative is present in a concentration of about 30 to 50 mg/mL, and present with a viscosity within the range 3.5 to 6.5 cP, and
c) a disaggregating agent, preferably sodium chloride, present in a concentration of about 0.5 to 2 mg/mL.

According to a more preferred embodiment of the present invention, the oral liquid pharmaceutical composition comprises;
a) salbutamol as an active agent or a pharmaceutically acceptable salt form,
b) a cellulose derivative, preferably HPMC, as thickening agent wherein the cellulose derivative is present in a concentration of 40 mg/mL, and present with a viscosity within the range 3.5 to 6.5 cP, and
c) a disaggregating agent, preferably sodium chloride, present in a concentration of about 0.5 to 2 mg/mL.

As a further embodiment of the present invention, the concentration ratio of thickening agent, preferably HPMC to disaggregating agent preferably sodium chloride is in the range of between 800:1 and 2:1 (mg/mL), preferably between 400:1 and 8:1. It has also been found that the combination of thickening agent with the disaggregating agent, particularly HPMC with sodium chloride, in the ratio suitable to the present invention, has a synergistic effect over the stability profile of the oral liquid composition.

According to a preferred embodiment of the present invention, the oral liquid pharmaceutical composition can contain a buffer system and a sweetening agent.

According to an embodiment of the present invention, the oral liquid pharmaceutical composition has a pH range of 3 to 5 provided by a buffer system including citrate buffers, phosphate buffers, or any other suitable buffer known in the art. The buffer system include preferably sodium citrate, potassium citrate, sodium citrate dihydrate, citric acid, citric acid monohydrate, sodium bicarbonate, potassium bicarbonate, sodium dihydrogen phosphate and potassium dihydrogen phosphate and the like or combination thereof. The most preferred buffer system of the present invention contains the combination of sodium citrate dihydrate and citric acid monohydrate. The concentration of buffer system used may be in the range of between 1 and 6 mg/mL and preferably between 3 ad 5 mg/mL.

According to an embodiment of the present invention, the oral liquid pharmaceutical composition comprises sweetening agent to enhances the taste of the composition. Sweetening agent used is selected from the group comprising, but not limited to, saccharine, saccharine sodium, sucralose, acesulfame potassium, aspartame, a fluorinated sucrose derivative, dextrose, corn syrup, alitame, cyclamic acid, thaumatin, monellin, monoammonium glycyrrhizinate and the like or combination therof. The most preferred sweetening agent is saccharine sodium. The concentration of sweetening agent used may be in the range of between 0.1 and 5 mg/mL, and preferably between 1.5 and 3mg/mL.

Accoding to an embodiment of the present invention, the oral liquid pharmaceutical composition can also contain other excipients commonly found in pharmaceutical compositions such as diluent, preservative and flavoring agent.

Diluent used according to the present invention is selected from the group comprising, but not limited to, water, dimethylisosorbide, glycols, ethyl alcohol, cetyl alcohol, glyceryl stearate, isopropyl alcohol, diethylamine, glyceryl oleate, myristyl alcohol, gelatin, cyclodextrin, polyvinyl pyrrolidone (Povidone), benzyl alcohol, glycerin, maltitol, xylitol, inositol, mannitol, invert sugars, sorbitol and the like or combination thereof. The preferred diluent is water. By this way, the composition of the present invention can be free of alcohol and can be suitable for administering to children.

Preservative used according to the present invention is selected from the group comprising, but not limited to, sodium benzoate, potassium sorbate, benzyl alcohol, parabens such as methylparaben, ethylparaben, propylparaben, butylparaben and the like or combination thereof. The most preferred preservative of the present invention is sodium benzoate. The concentration of preservatives used may be in the range of between 0.1 and 4 mg/mL and preferably between 1 and 3 mg/mL.

Flavoring agent used according to the present invention is selected from the group comprising but not limited to, orange, peppermint, spearmint, lime, apple, pear, peach, raspberry, plum, pineapple flavour and the like or combination thereof. The concentration of flavoring agent used may be in the range of between 0.1 and 2 mg/mL and preferably between 0.1 and 0.5 mg/mL.

In the present invention, to enhance the stability, solubility and processing time of oral liquid pharmaceutical composition, this composition has been designed, made up of the following:
a) salbutamol as an active agent or a pharmaceutically acceptable salt form,
b) HPMC as thickening agent in a concentration of 40mg/mL,
c) sodium chloride as disaggregating agent in a concentration of 1 mg/mL,
d) the combination of sodium citrate dihydrate and citric acid monohydrate as a buffer system in a concentration of 4.5 mg/mL,
e) saccharine sodium as sweetening agent in a concentration of 2 mg/mL,
f) sodium benzoate as preservative in a concentration of 2 mg/mL,
g) orange flavor as flavoring agent in a concentration of 0.2 mg/mL, and
h) pure water as diluent.

The oral liquid pharmaceutical composition of the present invention can be prepared by various methods. One embodiment of a manufacturing method is as follows:
a) purified water is heated to 60°C, while mixing by mechanical mixer,
b) mixing thickening agent with heated water,
c) cooling the mixture to 40ºC when a clear solution is obtained,
d) adding, to the mixture obtained at step c), salbutamol, disaggregating agent, buffer system, sweetening agent and preservative and mixing them while cooling to 30°C,
e) adding flavoring agent and mixing,
f) if necessary, adjusting the pH of the final mixture to the range 3 to 5.

## Claims

1. An oral liquid pharmaceutical composition comprising salbutamol as an active agent or a pharmaceutically acceptable salt form and a thickening agent as an excipient wherein the thickening agent is in a concentration of 20 to 80 mg/mL and has a viscosity within the range of from 1 to 10 cP.

2. The oral liquid pharmaceutical composition according to claim 1, wherein the thickening agent is in a concentration of 30 to 50 mg/mL.

3. The oral liquid pharmaceutical composition according to claim 2, wherein the thickening agent is in a concentration of 40mg/mL.

4. The oral liquid pharmaceutical composition according to claim 1, wherein the thickening agent has a viscosity within the range of from 3.5 to 6.5 cP.

5. The oral liquid pharmaceutical composition according to any preceding claims, wherein the thickening agent is selected from the group comprising cellulose derivatives, guar gum, gelatin, locust bean gum, tara gum, xanthan gum, tamaring gum, povidone, aluminium silicate, magnesium silicate, propylene glycol alginate, sodium carboxymethylcellulose, sodium alginate, pectin, carrageenan, polyethylene glycol, modified starch and combination thereof.

6. The oral liquid pharmaceutical composition according to claim 5, wherein the cellulose derivatives are cellulose hydroxypropyl cellulose, hydroxypropyl methylcellulose, hydroxyethyl cellulose, methyl cellulose, microcrystalline cellulose or combination thereof.

7. The oral liquid pharmaceutical composition according to claim 6, wherein the cellulose derivative is hydroxypropyl methylcellulose.

8. The oral liquid pharmaceutical composition according to any preceding claims, further comprising an additional disaggregating agent.

9. The oral liquid pharmaceutical composition according to claim 8, wherein the additional disaggregating agent is in a concentration of about 0.1 to 5 mg/mL or preferably in a concentration of about 0.5 to 2 mg/mL.

10. The oral liquid pharmaceutical composition according to claim 9, wherein the additional disaggregating agent is selected from the group comprising sodium chloride, calcium chloride, potassium chloride, calcium phosphate, calcium sulphate, starch, alginic acid, sodium starch glycolate, alginates, boric acid, sorbitol, glycerol and combination thereof.

11. The oral liquid pharmaceutical composition according to claim 10, wherein the additional disaggregating agent is sodium chloride.

12. The oral liquid pharmaceutical composition according to any preceding claims comprising,
a) salbutamol as an active agent or a pharmaceutically acceptable salt form,
b) hydroxypropyl methylcellulose as thickening agent which is in a concentration of 20 to 80 mg/mL and has a viscosity within the range of from 1 to 10 cP, and
c) sodium chloride as disaggregating agent in a concentration of about 0.1 to 5 mg/mL.

13. The oral liquid pharmaceutical composition according to claim 12 comprising,
a) salbutamol as an active agent or a pharmaceutically acceptable salt form,
b) hydroxypropyl methylcellulose as thickening agent which is in a concentration of 40 mg/mL and has a viscosity within the range of from 3.5 to 6.5 cP, and
c) sodium chloride as disaggregating agent in a concentration of about 0.5 to 2 mg/mL.

14. The oral liquid pharmaceutical composition according to claim 12 and 13, wherein the concentration ratio of hydroxypropyl methylcellulose to sodium chloride is in the range of between 800:1 and 2:1.

15. The oral liquid pharmaceutical composition according to claim 14, wherein the concentration ratio of hydroxypropyl methylcellulose to sodium chloride is in the range of between 400:1 and 8:1.
